Europäisches Patentamt

European Patent Office

Office européen des brevets

(11) Numéro de publication: **0 222 704**
**A2**

(12) **DEMANDE DE BREVET EUROPEEN**

(21) Numéro de dépôt: 86810503.2

(22) Date de dépôt: 04.11.86

(51) Int. Cl.⁴: **C 08 B 37/12**
C 08 H 5/02, C 12 N 1/00

(30) Priorité: 13.11.85 CH 4857/85

(43) Date de publication de la demande:
20.05.87 Bulletin 87/21

(84) Etats contractants désignés:
AT BE CH DE ES FR GB IT LI LU NL SE

(71) Demandeur: **MEDINATIONAL (HARLEY STREET) LIMITED**
17 Widegate Street
Londres E1 7HP (GB)

(72) Inventeur: **Hubacher, Ulrich**
Glärnich Strasse 9
CH-8132 Egg (CH)

(74) Mandataire: **Vuille, Roman et al**
c/o KIRKER & Cie S.A. 14, rue du Mont-Blanc Case Postale 872
CH-1211 Genève 1 (CH)

(54) Procédé de préparation d'un complexe de polygalactane macromoléculaire et de composé azoté et utilisation de ce complexe.

(57) On prépare un complexe anhydre neutre de polygalactane macromoléculaire et d'un composé azoté, destiné à la reconstitution d'un milieu de culture pour microorganismes contenant des substances nutritives azotées, en mélangeant à température ambiant, en proportions prédéterminées, un polygalactane macromoléculaire présentant une structure linéaire avec un composé azoté jusqu'à obtention d'un produit homogène, en laissant reposer le mélange obtenu puis en le séchant, en broyant ensuite et, le cas échéant, calibrant par tamisage le produit desséché.

EP 0 222 704 A2

**0 222 704**

**Description**

Procédé de préparation d'un complexe de polygalactane macromoléculaire et de composé azoté, et utilisation de ce complexe

L'invention a pour objet la préparation d'un nouveau complexe de polygalactane macromoléculaire et de composé azoté, destiné à la reconstitution de milieux de culture gélifiés, pour microorganismes, contenant des substances nutritives azotées. Elle a également pour objet l'utilisation de tels complexes.

En microbiologie, un très grand nombre de milieux de culture destinés à l'isolement des microorganismes (bactéries, levures, petits champignons, petits insectes, petits végétaux...) comporte un produit gélifiant qui est l'agar, substance inerte produite par des algues marines qui rend le milieu solide, ce qui est nécessaire à l'isolement en surface, ainsi que des substances nutritives d'origines diverses et accessoirement des substances réactives (colorants, indicateurs...).

Lors de leur utilisation, ces milieux de culture sont préparés à partir de produits vendus séparément, les produits sont mélangésetsolubilisés dans l'eau chaude pour faire fondre l'agar qui gélifie par refroidissement.

Des mélanges de poudres contenant l'agar et les substances nutritives desséchées sont déjà commercialisés. Il suffit d'en peser une certaine quantité et de faire fondre dans l'eau à chaud. Il s'agit toujours de simples mélanges de poudres.

Les exigences nutritives des microorganismes étant très variables et très complexes, on a pris l'habitude de leur fournir des substances nutritives complexes d'origine naturelle, en particulier des protéines plus ou moins hydrolysées pour les rendre plus facilement assimilables.

La normalisation de tels milieux est de ce fait difficile car ils comportent en règle générale, pour les 2/3 du poids environ, des produits d'origine naturelle qui sont chimiquement variables, tels l'agar et les hydrolysats de protéines par exemple. Les ingrédients de base se présentent en outre sous forme de poudres pouvant varier l'une de l'autre quant à leur hygrométrie, leur granulométrie ou leur stabilité chimique. On a même constaté, lors du fractionnement de mélanges terminés en plusieurs lots, que l'homogénéité des mélanges pulvérulents obtenus ne pouvait être toujours garantie, ceci même en partant d'agar purifié et, par exemple, de peptides purifiés et standardisés.

Il a été découvert que les inconvénients susmentionnés pouvaient être aisément surmontés à l'aide de complexes de polygalactanes macromoléculaires et de composés azotés, des peptides par exemple, tels qu'obtenus selon le procédé de la revendication 1. A l'aide d'un tel procédé en effet, on obtient un complexe stable, mécaniquement indissociable, que l'on peut avantageusement utiliser pour la reconstitution de tous les milieux de culture actuellement préparés à partir d'agar et de substances nutritives azotées.

De façon surprenante, on a observé que les complexes de la présente invention se comportaient différemment du simple mélange des mêmes ingrédients de base: les opérations de broyage et de tamisage ne modifient pas la composition du complexe, contrairement à celle du mélange; la stabilité de certaines substances azotées, acides aminés ou peptides par exemple est augmentée; la résistance au vieillissement est également augmentée, et après dissolution dans l'eau, la qualité des substances nutritives azotées demeure inchangée par rapport à celle des ingrédients de base entrant dans la composition des complexes selon l'invention.

Le procédé de la présente invention se caractérise par le fait qu'on mélange à température ambiante, en proportions prédéterminées, un polygalactane macromoléculaire présentant une structure linéaire avec un composé azoté jusqu'à obtention d'un produit homogène, on laisse reposer le mélange obtenu puis on le sèche, on broye ensuite et, le cas échéant, calibre par tamisage le produit desséché.

On obtient ainsi un complexe de polygalactane macromoléculaire et de composé azoté que l'on peut avantageusement utiliser pour la reconstitution d'un milieu de culture gélifié pour microorganismes contenant des substances nutritives azotées.

Selon l'invention, on utilise comme ingrédient de départ un polygalactane présentant une structure macromoléculaire linéaire. Par ces termes, on entend définir des macromolécules dont l'arrangement des chaînons de sucres qui les composent est quasi, sinon parfaitement, linéaire, à la différence de l'arrangement spatial des macromolécules d'agarose naturel à l'état sec ou gélifié, qui est tridimensionnel. Il est en effet connu qu'à l'état gélifié tout au moins, les molécules d'agarose (polygalactane) existent sous forme appariée, plus précisément sous forme de double hélice. De telles formes de conviennent pas à la mise en oeuvre du procédé de la présente invention.

Le polygalactane macromoléculaire de structure linéaire peut être par exemple obtenu à partir d'agarose naturel floculé et stabilisé conformément aux techniques usuelles. De façon générale, la floculation de l'agarose s'effectue par addition d'un solvant organique polaire à une solution aqueuse d'agarose maintenue en fusion à chaud. A titre de solvant organique, on utilise avantageusement le méthanol, l'éthanol, le propanol, l'isopropanol ou l'acétone par exemple,ou des mélanges de tels solvants. Une fois recueilli, l'agarose floculé est essoré puis stabilisé par addition d'une petite quantité (2 à 10% en poids) d'un solvant organique tel que ceux mentionnés ci-dessus. De l'agarose naturel floculé et stabilisé au moyen d'alcool éthylique convient parfaitement bien à la mise en oeuvre du procédé de la présente invention.

En règle générale, toute méthode connue de floculation et de stabilisation d'un polygalactane tel l'agarose naturel peut être utilisée, dès lors qu'elle conduit à l'obtention de macromolécules de structure linéaire.

Selon l'invention, on peut également utiliser des polygalactanes synthétiques ou semi-synthétiques dont les macromolécules possèdent la structure désirée. A ce titre, on peut citer le polygalactane de synthèse - oxyde tel que défini dans la demande de brevet FR 8303368 par exemple.

Selon l'invention, on utilise à titre de composé azoté, un composé azoté minéral ou organique. En guise de composé azoté minéral, on peut utiliser, selon les besoins, un nitrite ou un nitrate de métal alcalin, le nitrate de sodium ou potassium par exemple, ou encore en sel d'ammonium tel le nitrate ou le chlorure d'ammonium, le phosphate ou le sulfate de diammonium par exemple. En guise de composé azoté organique, on peut avantageusement utiliser un sel d'ammonium d'un acide organique tel le citrate, le tartrate ou l'acétate d'ammonium, ou une amine primaire, secondaire ou tertiaire par exemple. Selon la destination du complexe. on peut également utiliser un acide aminé ou un mélange d'acides aminés ou de leurs sels, tels le chlorhydrate de lysine ou de phénylalamine par exemple, ou encore un peptide ou un mélange de peptides, un polypeptide ou un mélange de polypeptides. A titre de peptide ou de mélanges de peptides, on peut citer entre autres des mélanges purifiés et standardisés obtenus à partir d'hydrolysats acides ou enzymatiques de protéines d'origines fort diverses, tels des hydrolysats de caséine du lait, de lactosérum ou de protéines de soja par exemple.

Le mélange des ingrédients choisis, pesés chacun en proportions prédéterminées, s'effectue au moyen des techniques usuelles, sans ajout de solvant. Selon les cas, en fonction de l'apport d'acide dû au composé azoté tel un chlorhydrate d'acide aminé, on incorpore au mélange en préparation la quantité d'alcali nécessaire à la neutralisation de l'acide ainsi introduit. On ajoute de préférance une base liquide relativement volatile, dont l'éventuel excès peut être aisément éliminé par évaporation, de l'ammoniaque de préférence.

A l'exception de l'alcali mentionné ci-dessus, les ingrédients utilisés, polygalactane et composé azoté, se présentent de préférence sous forme pulvérulente sèche, bien que cela ne représente pas une condition indispensable à la bonne mise en oeuvre du procédé de l'invention, en ce qui concerne le composé azoté.

Une fois le mélange intime des constituants réalisé, le produit homogène résultant est mis à reposer un certain temps, 12, 24 ou 48 h selon les cas, de façon que la complexation et l'éventuelle neutralisation soient les plus complètes possible. Tout comme le mélange proprement dit, cette phase de repos se déroule en règle générale à température ambiante. Le produit résultant est ensuite séché, de préférence sous pression réduite de façon à éliminer tout excès de produit volatil, et finalement broyé au moyen des techniques usuelles.

Le broyat sec ainsi obtenue peut être ensuite calibré par tamisage: cette opération a l'avantage de permettre l'obtention d'un complexe pulvérulent parfaitement homogène quant à sa granulométrie, ce qui facilitera d'autant son mélange avec des ingrédients additionnels, de granulométrie comparable, en vue de la préparation de milieux de culture. La phase ultime de tamisage présente en outre l'avantage de permettre d'éliminer par ce moyen un éventuel excès de composé azoté non complexé, la granulométrie des sels minéraux ou organiques, des acides aminés ou des peptides utilisés étant préalablement définie.

Grâce au procédé de la présente invention, on obtient des complexes pulvérulents neutres parfaitement homogènes, dont la composition en éléments azotés peut être définie avec précision. De tels complexes se sont révélés particulièrement stables dans des conditions normales de conservation; ils peuvent être en outre fractionnés en petits lots à tout moment, juste avant l'emploi par exemple. Après addition éventuelle de substances telles que colorants, indicateurs, électrolytes ou autres, dissolution à chaud dans une quantité prédéterminée d'eau (110°C ou plus), répartition de la solution sur des supports adéquats et refroidissement, on obtient un milieu de culture gélifié dont le contenu en substances nutritives azotées peut être déterminé avec précision.

On donnera ci-après quelques exemples de complexes selon l'invention que l'on peut proposer pour la reconstitution de milieux de cultures pour microorganismes (quantités données pour 1000 ml d'eau):
- chlorure d'ammonium 1,91 g
polygalactane 12,00 g
(azote assimilable 0,5 g/l)
- nitrate d'ammonium 2,86 g
polygalactane 12,00 g
(azote assimilable 1,0 g/l)
- diammonium phosphate 9,43 g
polygalactane 12,00 g
(azote assimilable 2,0 g/l)
- peptides ex-caséine 5,00 g
polygalactane 12,00 g
- peptides ex-lactosérum 10,00 g
polygalactane 12,00 g
- peptides ex-caséine et ex-lactosérum 10,00 g
polygalactane 12,00 g
- peptides ex-protéines de soja 5,00 g
polygalactane 12,00 g

De tels complexes conviennent parfaitement bien à la reconstitution de milieux nutritifs normalisés.

Les exemples suivants illustrent l'invention de façon plus détaillée, sans pour autant la limiter.

Exemple 1

Préparation d'un complexe d'agarose et de peptides de caséine

a. Floculation de l'agarose

70 g d'agar bactériologique ont été placés dans un réacteur de 5 l et additionnées de 1000 ml d'eau. Le mélange a été chauffé dans un autoclave durant 15 min env. à 110°C, jusqu'à obtention d'une solution limpide. Après refroidissement à 50-60°C, la solution a été versée dans un récipient contenant 5 l d'alcool éthylique pur, le produit de floculation ainsi obtenu s'accumulant au fond du récipient au fur et à mesure de sa formation.

Le floculat a été recueilli par filtration, broyé finement à l'aide d'un mélangeur-broyeur et finalement essoré. Le produit résultant a ensuite été séché en atmosphère d'alcool, en présence d'un piège à eau, jusqu'à obtention d'un résidu sec à plus de 90%. Le produit blanc pulvérulent ainsi obtenu a été stabilisé par addition d'alcool éthylique à raison de 5 g d'alcool pur pour 100 g de poudre et conservé dans un récipient étanche.

b. Préparation du complexe

10 g d'agarose floculé et stabilisé (résidu sec 9 g; voir lettre a ci-dessus) ont été mélangés à 0,9 ml d'ammoniaque à 25 vol. dans $H_2O$ et 15 g d'un hydrolysat acide de caséine contenant des peptides de poids moléculaire compris entre 300 et 1000. On a travaillé le mélange jusqu'à obtention d'un produit pâteux homogène et laissé ce produit reposer durant 24 heures avant de le sécher sous vide.

Le produit résultant a été finalement broyé et calibré par tamisage: on recueille ainsi une poudre blanc-crème, de pH neutre. Le contrôle du produit peut être effectué par mesure de la dureté du gel obtenu après dissolution dans l'eau et/ou dosage de l'azote total, par exemple.

Le complexe ainsi préparé sera caractérisé par son rapport agarose/peptides (poids à l'état sec): 9/15 dans le cas ci-dessus.

Ce complexe peut être utilisé comme suit pour la préparation d'un milieu de culture de type BCP (Bromocrésol-purple medium) convenant à la culture de bactéries urinaires:
- complexe d'agarose et de peptides de caséine 9/15 22 g
- lactose 10 g
- pourpre de bromocrésol 0,025 g

Après mélange des constituants, la quantité désirée est pesée et ajoutée à de l'eau (par ex: 32,025 g pour 1000 ml $H_2O$), chauffée durant 20 min. dans un autoclave à 110°C et finalement répartie dans des boîtes de Petri.

Exemple 2

Préparation d'un complexe d'agarose et de lysine

10 g d'agarose floculé et stabilisé selon le procédé de l'exemple 1 (letter a) et 1 g de chlorhydrate de lysine ont été mélangés en présence de la quantité d'ammoniaque nécessaire à la neutralisation de l'acide chlorhydrique introduit par le chlorhydrate, jusqu'à obtention d'une masse pâteuse homogène.

Après entreposage durant 24 h à température ambiante et dessication sous vide, le produit résultant a été broyé et tamisé jusqu'à obtention de la qualité désirée.

L'analyse effectuée montre que dans le complexe ainsi obtenu, 99% de l'azote dosé se trouve sous forme de lysine et 99% de la lysine complexée se présente sous forme monomoléculaire (poids moléculaire 146).

Exemple 3

Préparation d'un milieu de culture à base de complexes agarose-peptides

On a préparé un premier complexe "agarose-peptides de caséine 6/10" à partir d'agarose floculé et d'hydrolysat acide de caséine selon le procédé de l'exemple 1.

On a préparé un second complexe "agarose-peptides de soja 6/3" à partir d'agarose floculé et d'hydrolysat acide, de protéines de soja, selon le procédé de l'exemple 1 également.

Après dessication sous vide et broyage, chacun des complexes obtenus a été calibré par tamisage de façon à recueillir un produit pulvérulent présentant la même granulométrie que le chlorure de sodium utilisé en bactériologie.

On a finalement préparé le milieu de culture désiré comme suit:

| | |
|---|---|
| - complexe "agarose-peptides de caséine 6/10" | 16 g |
| - complexe "agarose-peptides de soja 6/3" | 9 g |
| - chlorure de sodium | 5 g |
| Total | 30 g |

La quantité de mélange obtenue est ajoutée à 1000 ml d'eau, puis chauffée à env. 110°C dans un autoclave

4

0 222 704

jusqu'à dissolution complète et finalement répartie dans des boîtes de Petri.

Etant donné l'homogénéité du mélange pulvérulent (granulométrie), celui-ci peut être divisé en plus petites portions sans que sa composition varie, notamment sa teneur en azote peptidique.

## Revendications

1. Procédé pour la préparation d'un complexe anhydre neutre de polygalactane macromoléculaire et de composé azoté, destiné à la reconstitution d'un milieu de culture pour microorganismes contenant des substances nutritives azotées, caractérisé en ce qu'on mélange à température ambiante, en proportions prédéterminées, un polygalactane macromoléculaire présentant une structure linéaire avec un composé azoté jusqu'à obtention d'un produit homogène, on laisse reposer le mélange obtenu puis on le sèche, on broye ensuite et, le cas échéant, calibre par tamisage le produit desséché.

2. Procédé selon la revendication 1, caractérisé en ce que le polygalactane macromoléculaire est de l'agarose floculé et stabilisé.

3. Procédé selon la revendication 1, caractérisé en ce que le polygalactane macromoléculaire est un polygalactane synthétique ou semi-synthétique tel le polygalactane de synthèse-oxyde.

4. Procédé selon la revendication 1, caractérisé en ce que le composé azoté est un composé azoté minéral tel un nitrite ou un nitrate de métal alcalin ou un sel d'ammonium d'un acide minéral.

5. Procédé selon la revendication 1, caractérisé en ce que le composé azoté est un composé azoté organique tel un sel d'ammonium d'un acide organique, une amine primaire, secondaire ou tertiaire, un acide aminé ou un mélange d'acides aminés, un sel d'acide aminé ou un mélange de sels d'acides aminés, un peptide ou un mélange de peptides, un polypeptide ou un mélange de polypeptides.

6. Procédé selon la revendication 5, caractérisé en ce que le composé azoté organique est un hydrolysat de protéines tel un hydrolysat acide ou enzymatique de caséine ou de protéines de soja.

7 Procédé selon l'une des revendications 1 à 6, caractérisé en ce que le mélange s'effectue le cas échéant, en présence de quantités stoechiométriques d'un agent basi que destiné à neutraliser l'acidité introduite par le composé azoté.

8. Procédé selon l'une des revendications 1 à 7, caractérisé en ce que le composé azoté se présente sous une forme pulvérulente sèche.

9. Procédé selon l'une des revendications 1 à 8, caractérisé en ce que l'excès de composé azoté non complexé est éliminé lors de la phase de calibrage par tamisage.

10. Complexe anhydre neutre de polygalactane macromoléculaire et de composé azoté tel qu'obtenu au moyen du procédé selon l'une des revendications 1 à 9.

11. Utilisation du complexe anhydre neutre de polygalactane macromoléculaire et de composé azoté selon la revendication 10 pour la reconstitution d'un milieu de culture gélifié pour microorganismes.